(19) Europäisches Patentamt
European Patent Office
Office européen des brevets

(11) **EP 3 929 229 A1**

(12) **EUROPEAN PATENT APPLICATION**
published in accordance with Art. 153(4) EPC

(43) Date of publication:
**29.12.2021 Bulletin 2021/52**

(51) Int Cl.:
*C08G 18/08* (2006.01)     *A61K 8/87* (2006.01)
*A61Q 5/06* (2006.01)

(21) Application number: **20758579.5**

(22) Date of filing: **17.02.2020**

(86) International application number:
**PCT/JP2020/005985**

(87) International publication number:
**WO 2020/171002 (27.08.2020 Gazette 2020/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.02.2019  JP 2019027294**

(71) Applicant: **ADEKA CORPORATION**
**Arakawa-ku**
**Tokyo**
**116-8554 (JP)**

(72) Inventors:
• **MIYAZONO, Keitarou**
  **Tokyo 116-8554 (JP)**
• **MATSUKURA, Noriyoshi**
  **Tokyo 116-8554 (JP)**
• **TSUSHIMA, Yasuhiro**
  **Tokyo 116-8554 (JP)**
• **SHIRAI, Hiroaki**
  **Tokyo 116-8554 (JP)**

(74) Representative: **Müller-Boré & Partner**
**Patentanwälte PartG mbB**
**Friedenheimer Brücke 21**
**80639 München (DE)**

(54) **POLYURETHANE FOR COSMETIC PREPARATIONS AND METHOD FOR PRODUCING POLYURETHANE FOR COSMETIC PREPARATIONS**

(57)     This invention provides: a polyurethane for cosmetic preparations, which is obtained by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group and two hydroxyl groups in the molecule and has a molecular weight of 100 to 300, (D) a diisocyanate containing at least one compound selected from the group consisting of isophorone diisocyanate, hexamethylene diisocyanate and dicyclohexylmethane-4,4'-diisocyanate, and (E) a chain extender which contains at least one compound selected from the group consisting of water, ethylene diamine and propylene diamine and which has a weight average molecular weight of 50,000 to 300,000; a production method; and a cosmetic preparation which contains this polyurethane for cosmetic preparations.

## Description

Technical Field

This invention relates to a polyurethane for cosmetic preparations, which can be advantageously used in cosmetic preparations, and to a method for producing a polyurethane for cosmetic preparations, which can be advantageously used in cosmetic preparations.

Background Art

Particles and coating films of polyurethanes exhibit flexibility, elasticity, stability, safety, and the like, and polyurethanes having a variety of structures have been developed and used in cosmetic preparations in the past. For example, Patent Literature (PTL 1) proposes an organic fine powder-containing cosmetic which contains a specific spherical polyurethane fine powder and which exhibits excellent slipperiness and feel on skin. In addition, PTL 2 proposes an aerosol composition which consists of a liquefied gas and an aqueous stock solution containing a urethane resin and which is useful as an aerosol type gommage cosmetic. Furthermore, PTL 3 proposes using a polyurethane salt, which consists of a poly(lactic acid) polyol, a diol and a diisocyanate, as an aid for a cosmetic or a pharmaceutical agent.

In addition, as an invention that utilizes the feel and coating characteristics of polyurethanes in particular, PTL 4 proposes a hair cosmetic, wherein by incorporating a chitosan compound and a polyurethane resin in the hair cosmetic, it is possible to impart tension and elasticity to thin and soft hair that has been damaged, enable supple hair with smooth finger-passing and no coarse feeling, suppress stickiness and roughness, and impart hair with a favorable texture. PTL 5 proposes a hair cosmetic which contains a cationic surfactant, an aliphatic alcohol, a polyurethane resin, a non-ionic surfactant, and the like, is excellent in terms of producing natural volume of parietal hair, softness of the hair and non-stiffness of the hair with respect to hair whose quality has changed as a result of aging, can be easily spread at the time of application, and enables hair to be styled easily. However, conventional polyurethanes are inadequate in terms of a balance among coating film flexibility, strength and stability, and there is a need for a polyurethane that simultaneously satisfies characteristics required when used in a cosmetic.

Citation List

Patent Literature

[PTL 1] Japanese Patent Application Publication No. H05-262622
[PTL 2] Japanese Patent Application Publication No. 2003-137730
[PTL 3] Japanese Translation of PCT Application No. WO H07-509741
[PTL 4] Republished International Patent Publication No. WO 2008/035563
[PTL 5] Japanese Patent Application Publication No. 2016-117715

Summary of Invention

Technical Problem

Therefore, the purpose of this invention is to provide: a polyurethane for cosmetic preparations, which exhibits excellent properties such as flexibility, strength and stability when used in a cosmetic preparation; and a method for producing a polyurethane for cosmetic preparations, which can be advantageously used in cosmetic preparations.

Solution to Problem

As a result of diligent research, the inventors of this invention found a polyurethane for cosmetic preparations, which exhibits excellent properties when used in cosmetic preparations, and thereby completed this invention. That is, this invention is a polyurethane for cosmetic preparations, which is obtained by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group and two hydroxyl groups in the molecule and has a molecular weight of 100 to 300, (D) a diisocyanate containing at least one compound selected from the group consisting of isophorone diisocyanate, hexamethylene diisocyanate and dicyclohexylmethane-4,4'-diisocyanate, and (E) a chain extender which contains at least one compound selected from the group consisting of water, ethylene diamine and propylene diamine and which has a weight average molecular weight of 50,000 to 300,000.

Advantageous Effects of Invention

The polyurethane for cosmetic preparations of this invention exhibits excellent properties such as flexibility, strength and stability when used in cosmetic preparations.

Description of Embodiments

The polyester polyol (A) having two or more hydroxyl groups in each molecule used in this invention can be a well-known polyester polyol, and examples thereof include compounds obtained by subjecting a polycarboxylic acid and a low molecular weight polyol to an esterification reaction and compounds obtained by subjecting a cyclic ester compound such as ε-caprolactone or γ-valerolactone to a ring opening polymerization reaction. In addition, the weight average molecular weight of the polyester polyol (A) is not particularly limited, but from the perspective of achieving a balance of properties in an obtained polyurethane for cosmetic preparations, this weight average molecular weight is, for example, preferably 200 to 10,000, more preferably 400 to 5,000, and further preferably 600 to 3,000. Moreover, weight average molecular weights in this invention can be measured by means of gel permeation chromatography (GPC) and determined in terms of styrene.

The upper limit for the number of hydroxyl groups per molecule of the polyester polyol (A) is not particularly limited, but is preferably 4, more preferably 3, and most preferably 2. It is most preferable for one hydroxyl group to be present at each terminal of the polyester polyol (A).

Examples of polycarboxylic acids include aliphatic polycarboxylic acids such as succinic acid, adipic acid, suberic acid, azelaic acid, sebacic acid, dodecane dicarboxylic acid and dimer acids; alicyclic polycarboxylic acids such as 1,4-cyclohexane dicarboxylic acid and cyclohexane tricarboxylic acid; aromatic polycarboxylic acids such as phthalic acid, isophthalic acid, terephthalic acid, 1,4-naphthalene dicarboxylic acid, 2,3-naphthalene dicarboxylic acid, 2,6-naphthalene dicarboxylic acid, biphenyl dicarboxylic acid, trimellitic acid and pyromellitic acid; and anhydrides and ester derivatives of these, and it is possible to use one of these or a combination of two or more types thereof.

Examples of low molecular weight polyols include aliphatic polyols such as ethylene glycol, diethylene glycol, triethylene glycol, tetraethylene glycol, propane diol, dipropylene glycol, tripropylene glycol, butane diol, pentane diol, hexane diol, heptane diol, octane diol, nonane diol, decane diol, undecane diol, dodecane diol, 2-methyl-1,3-propane diol, neopentyl glycol, hexylene glycol, 2-butyl-2-ethyl-1,3-propane diol, 3-methyl-1,5-pentane diol, 2-ethyl-1,3-hexane diol, 2-methyl-1,8-octane diol, glycerin, trimethylolpropane, ditrimethylolpropane, tritrimethylolpropane and pentaerythritol; and aliphatic ring type structure-containing polyols such as 1,4-cyclohexanedimethanol and hydrogenated bisphenol A, and it is possible to use one of these or a combination of two or more types thereof.

From the perspective of achieving a balance of properties in the polyurethane for cosmetic preparations of this invention, the polyester polyol (A) is preferably a polyester polyol obtained by reacting one or more types of polycarboxylic acid selected from the group consisting of phthalic acid, isophthalic acid, terephthalic acid and adipic acid with one or more types of aliphatic polyol selected from the group consisting of ethylene glycol, diethylene glycol, butane diol, neopentyl glycol, hexane diol and hexylene glycol, and is particularly preferably a polyester polyol obtained by reacting one or more types selected from the group consisting of phthalic acid, isophthalic acid and terephthalic acid with adipic acid and hexane diol.

Examples of the aliphatic diol (B) consisting of two hydroxyl groups and an aliphatic hydrocarbon group having 3 to 8 carbon atoms used in this invention include propane diol, butane diol, pentane diol, hexane diol, heptane diol, octane diol, 2-methyl-1,3-propane diol, neopentyl glycol, 3-methyl-1,5-pentane diol and 2-ethyl-1,3-hexane diol. Among such compounds, use of an aliphatic diol consisting of two hydroxyl groups and an aliphatic hydrocarbon group having 4 to 6 carbon atoms as the aliphatic diol (B) is preferred from the perspective of achieving a balance of properties in the polyurethane for cosmetic preparations of this invention, and more specifically, use of at least one type selected from among butane diol, pentane diol, hexane diol, 2-methyl-1,3-propane diol, neopentyl glycol and 3-methyl-1,5-pentane diol is preferred, use of at least one type selected from among hexane diol, 2-methyl-1,3-propane diol and neopentyl glycol is more preferred, and use of neopentyl glycol is further preferred.

The carboxyl group-containing diol (C) used in this invention, which has a carboxyl group and two hydroxyl groups in the molecule and has a molecular weight of 100 to 300, is not particularly limited, but examples thereof include dimethylolpropionic acid, dimethylolbutanoic acid, dimethylolbutyric acid and dimethylolvaleric acid. Of these, use of dimethylolpropionic acid as the carboxyl group-containing diol (C) is preferred from the perspective of achieving a balance of properties in the polyurethane for cosmetic preparations of this invention.

The diisocyanate (D) used in this invention includes at least one type selected from the group consisting of isophorone diisocyanate, hexamethylene diisocyanate and dicyclohexylmethane-4,4'-diisocyanate. By using this type of compound as the diisocyanate, the polyurethane for cosmetic preparations of this invention can exhibit excellent coating film flexibility, strength and stability. From the perspective of achieving a balance of properties in the polyurethane for cosmetic preparations of this invention, it is preferable to incorporate isophorone diisocyanate as the diisocyanate (D).

Moreover, other diisocyanate compounds may be used in this invention as long as the advantageous effect of this invention is not impaired, and examples thereof include tetramethylene diisocyanate, pentamethylene diisocyanate, 2,2-dimethylpentane diisocyanate, 3-methoxyhexane diisocyanate, octamethylene diisocyanate, 2,2,4-trimethylpentane diisocyanate, nonamethylene diisocyanate, decamethylene diisocyanate, 3-butoxyhexane diisocyanate, dodecamethylene diisocyanate; cyclohexane diisocyanate, methylcyclohexane diisocyanate, ethylcyclohexane diisocyanate, propylcyclohexane diisocyanate, dicyclohexylmethane-2,2'-diisocyanate, dicyclohexylmethane-2,4'-diisocyanate, toluidene diisocyanate, meta-phenylene diisocyanate, para-phenylene diisocyanate, dimethylbenzene diisocyanate, ethylbenzene diisocyanate, isopropylbenzene diisocyanate, meta-xylylene diisocyanate, para-xylylene diisocyanate, 1,4-naphthalene diisocyanate, 1,5-naphthalene diisocyanate, 2,6-naphthalene diisocyanate and 2,7-naphthalene diisocyanate.

From the perspective of achieving a balance of properties in the polyurethane for cosmetic preparations of this invention, the molar ratio of the total amount of isophorone diisocyanate, hexamethylene diisocyanate and dicyclohexylmethane-4,4'-diisocyanate and the amount of other diisocyanate compounds in the diisocyanate (D) is preferably 1-0.5:0-0.5 (the sum of the molar ratios is 1), and more preferably 1-0.8:0-0.2 (the sum of the molar ratios is 1), and it is further preferable not to use other diisocyanate compounds.

The chain extender (E) used in this invention includes at least one type selected from the group consisting of water, ethylenediamine and propylenediamine, and it is preferable for the chain extender (E) to consist of at least one type selected from the group consisting of water, ethylenediamine and propylenediamine. By using this type of compound as the chain extender, the polyurethane for cosmetic preparations of this invention can exhibit excellent coating film flexibility, strength and stability. Moreover, the chain extender in this invention functions as a chain extender by being added to/reacted with another material in a state whereby an isocyanate group able to react with the chain extender is present.

The weight average molecular weight of the polyurethane for cosmetic preparations of this invention is 50,000 to 300,000. By setting the weight average molecular weight to fall within this range, the polyurethane for cosmetic preparations of this invention can exhibit excellent coating film flexibility, strength and stability. From the perspective of achieving a balance of properties in the polyurethane for cosmetic preparations, this weight average molecular weight is preferably 60,000 to 250,000, more preferably 80,000 to 200,000, and further preferably 100,000 to 160,000. Weight average molecular weights in this invention can be measured by means of gel permeation chromatography (GPC) and determined in terms of styrene.

The polyurethane for cosmetic preparations of this invention can be obtained by reacting components (A) to (E) described above. The polyurethane for cosmetic preparations of this invention can be advantageously obtained by reacting only components (A) to (E) described above, that is, reacting materials consisting of components (A) to (E) described above. In another embodiment, the polyurethane for cosmetic preparations of this invention can be obtained by reacting materials consisting of components (A) to (E) described above and a neutralizing agent that is described later. First, a prepolymer is formed by forming urethane bonds through reactions between hydroxyl groups contained in components (A) to (C) and isocyanate groups contained in component (D). However, because the diisocyanate (D) and compounds having hydroxyl groups react in an arbitrary manner, it is impossible to unambiguously describe the structure of the prepolymer by means of a general formula, and it is therefore impossible to unambiguously describe the structure of the polyurethane for cosmetic preparations of this invention by means of a general formula. In addition, the speed of the reaction between the hydroxyl groups and the isocyanate group is rapid, there is no need to consider esterification reactions between the hydroxyl groups and carboxyl groups in the carboxyl group-containing diol (C) having a molecular weight of 100 to 300, and such esterification reactions have no effect on the structure of the polyurethane for cosmetic preparations of this invention.

The polyurethane for cosmetic preparations of this invention can be used in well-known cosmetic preparations, and examples of cosmetic preparations include skin lotions, cosmetic liquids, milky lotions, creams, face washing foams, cleansing milks, cleansing lotions, hair tonics, hair liquids, setting lotions, hair bleaches, color rinses, permanent wave liquids, lipsticks, beauty packs, foundations, eaux de cologne, shampoos, conditioners, treatments, sunscreens, deodorants, perfumes, cleansing oils and cosmetic oils.

The blending quantity of the polyurethane for cosmetic preparations of this invention in a cosmetic preparation is not particularly limited, and can be adjusted in view of conditions such as the type of cosmetic preparation in which the polyurethane is to be blended, but may be, for example, 0.01 to 50 mass% relative to the total quantity of the cosmetic preparation.

In addition, the method for adding the polyurethane for cosmetic preparations of this invention to a cosmetic preparation is not particularly limited, and well-known methods can be used, an example of which is a method comprising adding the polyurethane for cosmetic preparations to a mixture obtained by mixing some or all of the other components of a cosmetic preparation and, if necessary, stirring and the like. The polyurethane for cosmetic preparations of this invention has the structure described above, and can therefore simultaneously improve a variety of properties when added to a cosmetic preparation.

This invention also encompasses a cosmetic preparation that contains the polyurethane for cosmetic preparations described above. Examples of the cosmetic preparations mentioned above include skin lotions, cosmetic liquids, milky lotions, creams, face washing foams, cleansing milks, cleansing lotions, skin mists, hair tonics, hair liquids, setting lotions, hair bleaches, color rinses, permanent wave liquids, lipsticks, beauty packs, foundations, eaux de cologne, shampoos, conditioners, treatments, sunscreens, deodorants, perfumes, cleansing oils and cosmetic oils. The state of the cosmetic preparation of this invention should be adjusted as appropriate according to the intended use or application thereof, and examples thereof include liquids, milky lotions, gels, creams, solid powders, foams and mists.

The content of the polyurethane for cosmetic preparations in the cosmetic preparation of this invention is not particularly limited, and can be adjusted in view of the intended use or application thereof, but may be, for example, 0.01 to 50 mass% relative to the total quantity of the cosmetic preparation.

In addition to the polyurethane for cosmetic preparations mentioned above, the cosmetic preparation of this invention can contain components commonly used in cosmetic compositions in order to improve or modify a variety of characteristics of the cosmetic composition during storage, during use or after use according to the intended use or application of the cosmetic composition (for example, solubility, dispersibility, stability, feeling of use, applicability, permeability, moisturizing properties, safety, aesthetic properties, optical characteristics, aromatic properties and whitening properties). Examples of such components include powder components, liquid oils/fats, solid oils/fats, waxes, higher alcohols, higher fatty acids, anionic surfactants, cationic surfactants, amphoteric surfactants, non-ionic surfactants, moisturizing agents, polymer compounds, metal ion sequestering agents, lower alcohols, polyhydric alcohols, sugars, amino acids and derivatives thereof, organic amines, pH adjusting agents, antioxidants, preservatives, blood circulation promoters, antiphlogistic agents, activators, whitening agents, antiseborrheic agents, antiinflammatory agents and a variety of extracts and plant seaweed extracts, and one or more types of these can be blended according to need.

A cosmetic preparation that contains the polyurethane for cosmetic preparations of this invention is preferably a hair treatment cosmetic preparation from the perspectives of coating film flexibility, strength and stability. Hair treatment cosmetic preparations are not particularly limited, but examples thereof include cosmetic preparations for hair care, eyebrows and eyelashes, and specific examples thereof include hair dyes, hair perming agents, shampoos, hair rinses, conditioners, hair treatment agents, hair creams, hair gels, hair lotions, hair mousses, hair oils, hair styling agents, hair curling agents, hair straightening agents, hair curling gels, hair tonics, eyebrow pencils, eyebrow mascaras, eyebrow powders, mascaras and eyeliners. In addition, the cosmetic preparation of this invention can be a hair cosmetic preparation for styling hair. In addition, the state of the hair treatment cosmetic preparation should be selected as appropriate in view of conditions such as the type of cosmetic preparation, and examples thereof include liquids, milky lotions, gels, creams, solid powders, foams and mists.

The content of the polyurethane for cosmetic preparations of this invention in a hair treatment cosmetic preparation that contains the polyurethane for cosmetic preparations is not particularly limited, but from the perspective of achieving a balance of properties is, for example, 0.1 to 30 mass%, more preferably 0.2 to 20 mass%, and most preferably 0.5 to 15 mass%, relative to the total amount of the hair treatment cosmetic preparation.

A hair treatment cosmetic preparation that contains the polyurethane for cosmetic preparations of this invention can contain other components commonly used in hair treatment cosmetic preparations. Examples of these other components include cationic surfactants, anionic surfactants, amphoteric surfactants, non-ionic surfactants, hydrocarbon oils, silicone oils, ester oils, higher alcohols, polyhydric alcohols, sugars and derivatives thereof, pH-adjusting agents, dyes and pigments, fragrances, ultraviolet radiation absorbers, solvents and components commonly used in cosmetic compositions mentioned above, and it is possible to use one or more of these.

Examples of cationic surfactants include lauryltrimethylammonium chloride, cetyltrimethylammonium chloride, stearyltrimethylammonium chloride, alkyltrimethylammonium chlorides, distearyldimethylammonium chloride, stearyltrimethylammonium saccharin, cetyltrimethylammonium saccharin, behenyltrimethylammonium methyl sulfate, behenyldimethylamine, diethylaminoethylamide behenate, dimethylaminopropylamide behenate, dimethylaminoethylamide behenate, stearyldimethylamine, palmitoxypropyldimethylamine and stearoxypropyldimethylamine, and it is possible to use one or more of these. The concentration of the cationic surfactant is preferably 0.001 to 10 mass%, and more preferably 0.01 to 5 mass%, relative to the total quantity of the hair treatment cosmetic preparation.

Examples of anionic surfactants include alkyl ether sulfate salts, alkyl sulfate salts, alkyl ether sulfate ester salts, alkenyl ether sulfate salts, alkenyl sulfate salts, olefin sulfonate salts, alkane sulfonate salts, saturated and unsaturated fatty acid salts, alkyl or alkenyl ether carboxylate salts, α-sulfo fatty acid salts, N-acylamino acid type surfactants, phosphoric acid monoester and diester type surfactants, sulfosuccinic acid esters, N-alkyloylmethyltaurine salts and derivatives of these, and specific examples of counter ions for anion groups include sodium ions, potassium ions and triethanolamine, and it is possible to use one or more of these. The concentration of the anionic surfactant is preferably 0.001 to 10 mass%, and more preferably 0.01 to 5 mass%, relative to the total quantity of the hair treatment cosmetic preparation.

Examples of amphoteric surfactants include betaine type amphoteric surfactants such as coconut oil fatty acid amidopropyldimethylacetic acid betaine, lauryldimethylamino acid betaine, 2-alkyl-N-carboxymethyl-N-hydroxymethylimidazolinium betaines, laurylhydroxysulfo betaine, lauroylamidoethylhydroxyethylcarboxymethyl betaine and metal salts of hydroxypropyl phosphoric acid; amino acid type amphoteric surfactants such as metal salts of β-laurylaminopropionic acid; sulfuric acid ester type amphoteric surfactants; and sulfonic acid type amphoteric surfactants, and it is possible to use one or more of these. The concentration of the amphoteric surfactant is preferably 0.001 to 10 mass%, and more preferably 0.01 to 5 mass%, relative to the total quantity of the hair treatment cosmetic preparation.

Examples of non-ionic surfactants include POE cetyl ether (ceteth), POE stearyl ether (steareth), POE behenyl ether, POE oleyl ether (oleth), POE lauryl ether (laureth), POE octyl dodecyl ether, POE hexyl decyl ether, POE isostearyl ether, POE nonyl phenyl ether, POE octyl phenyl ether, POE polyoxypropylene cetyl ether, POE polyoxypropylene decyl tetradecyl ether, POE sorbitan monooleate, POE sorbitan monostearate, POE sorbitan monopalmitate, POE sorbitan monolaurate, POE sorbitan trioleate, POE glycerin monostearate, POE glycerin monomyristate, POE sorbitol tetraoleate, POE sorbitol hexastearate, POE sorbitol monolaurate, POE sorbitol beeswax, polyethylene glycol monooleate, polyethylene glycol monostearate, polyethylene glycol monolaurate, lipophilic glycerin monooleate, lipophilic glycerin monostearate, self-emulsifying glycerin monostearate, sorbitan monooleate, sorbitan sesquioleate, sorbitan trioleate, sorbitan monostearate, sorbitan monopalmitate, sorbitan monolaurate, sucrose fatty acid esters, decaglyceryl monolaurate, decaglyceryl monostearate, decaglyceryl monooleate, decaglyceryl monomyristate, alkyl glucosides, POE methyl glucoside and POE methyl glucoside dioleate, and it is possible to use one or more of these. The concentration of the non-ionic surfactant is preferably 0.001 to 10 mass%, and more preferably 0.01 to 5 mass%, relative to the total quantity of the hair treatment cosmetic preparation.

Examples of hydrocarbon oils include liquid paraffin, squalane, pristane, ozokerite, paraffins, ceresin, Vaseline, polyisobutene, polyisoprene, isodecane, isododecane, isohexadecane, n-pentane, isopentane, n-hexane, isohexane, kerosene, decalin, tetralin and microcrystalline waxes, and it is possible to use one or more of these. The concentration of the hydrocarbon oil is preferably 0.1 to 50 mass%, and more preferably 0.5 to 30 mass%, relative to the total quantity of the hair treatment cosmetic preparation.

Examples of silicone oils include chain-like silicone oils such as dimethylpolysiloxane, diphenylpolysiloxane, diphenylsiloxyphenyltrimethicone and octamethyltrisiloxane; cyclic silicone oils such as decamethylcyclotetrasiloxane, dodecamethylcyclotetrasiloxane, octamethylcyclotetrasiloxane, cyclopentasiloxane, dodecamethylcyclopentasiloxane, octamethylcyclopentasiloxane, decamethylcyclohexasiloxane, dodecamethylcyclohexasiloxane and octamethylcyclohexasiloxane; and modified silicone oils such as alkyl-modified dimethylpolysiloxanes, polyether-modified dimethylpolysiloxanes, fatty acid-modified polysiloxanes, higher alcohol-modified polysiloxanes, amino-modified polysiloxanes and fluorine-modified polysiloxanes, and it is possible to use one or more of these. The concentration of the silicone oil is preferably 0.1 to 50 mass%, and more preferably 0.5 to 30 mass%, relative to the total quantity of the hair treatment cosmetic preparation.

Examples of ester oils include synthetic ester oils such as ethyl acetate, butyl acetate, hexyl acetate, decyl acetate, butyl propionate, cetyl octanoate, hexyldecyl dimethyloctanoate, isononyl isononanoate, isononyl isononanoate, isotridecyl isononanoate, ethyl laurate, hexyl laurate, myristyl myristate, isopropyl myristate, isopropyl palmitate, 2-ethylhexyl palmitate, 2-hexyldecyl palmitate, 2-heptylundecyl palmitate, decyl oleate, oleyl oleate, octyldodecyl oleate, isocetyl stearate, glyceryl stearate, butyl stearate, ethylhexyl hydroxystearate, ethylene glycol stearate, octyl oxystearate, diethyl phthalate, triethyl citrate, 2-ethylhexyl succinate, diisopropyl sebacate, di-2-ethylhexyl sebacate, cetyl lactate, myristyl lactate, diisobutyl adipate, 2-hexyldecyl adipate, di-2-heptylundecyl adipate, cetyl ethyl hexanoate, triethylhexanoin, polyglyceryl-2 triisostearate, neopentyl glycol dioctanoate, neopentyl glycol dicaprate and pentaerythrityl tetraisostearate; and plant-based and animal-based ester oils such as lanolin, mink oil, cocoa butter, coconut oil, palm kernel oil, camellia oil, sesame oil, castor oil and olive oil, and it is possible to use one or more of these. The concentration of the ester oil is preferably 0.1 to 50 mass%, and more preferably 0.5 to 30 mass%, relative to the total quantity of the hair treatment cosmetic preparation.

Examples of higher alcohols include cetyl alcohol, isostearyl alcohol, lauryl alcohol, hexadecyl alcohol and octadecyl alcohol, and it is possible to use one or more of these. The concentration of the higher alcohol is preferably 0.1 to 30 mass%, and more preferably 0.5 to 20 mass%, relative to the total quantity of the hair treatment cosmetic preparation.

Examples of polyhydric alcohols include ethylene glycol, diethylene glycol, triethylene glycol, polyethylene glycol, highly polymerized polyethylene glycol, propylene glycol, dipropylene glycol, polypropylene glycol, isoprene glycol, 1,3-butylene glycol, glycerin, diglycerol and polyglycerol, and it is possible to use one or more of these. The concentration of the polyhydric alcohol is preferably 0.1 to 30 mass%, and more preferably 0.5 to 20 mass%, relative to the total quantity of the hair treatment cosmetic preparation.

Examples of sugars and derivatives thereof include xylose, D-glucose, sucrose, trehalose, fructose, maltose, mannose, cyclodextrin, β-glucan, chitin, chitosan, pectin, arabinogalactan, dextrin, dextran, and polymers and copolymers of glucosylethyl methacrylate, and it is possible to use one or more of these. The concentration of the sugar or derivative thereof is preferably 0.001 to 10 mass%, and more preferably 0.01 to 5 mass%, relative to the total quantity of the hair treatment cosmetic preparation.

Examples of pH-adjusting agents include citric acid, glycolic acid, succinic acid, tartaric acid, lactic acid, malic acid, levulinic acid, acetic acid, butyric acid, valeric acid, oxalic acid, maleic acid, fumaric acid, mandelic acid, phosphoric acid, pyrophosphoric acid, hydrochloric acid, sulfuric acid and nitric acid, and it is possible to use one or more of these. The pH-adjusting agent is preferably added so that the pH of the hair treatment cosmetic preparation of this invention falls within the range 3.0 to 13.0.

Examples of dyes and pigments include legal colorants, acidic dyes, basic dyes, oxidation dye intermediates, couplers, autoxidation type dyes, nitro dyes, disperse dyes, inorganic pigments, metal powder pigments and products which are obtained by surface treating these dyes and pigments, and it is possible to use one or more of these. The concentration of the dye or pigment is preferably 0.001 to 10 mass%, and more preferably 0.01 to 5 mass%, relative to the total quantity of the hair treatment cosmetic preparation.

Examples of fragrances include acetyl cedrene, allyl amyl glycolate, β-ionone, isobutyl quinoline, iris oil, irones, indole, undecanal, undecenal, γ-undecalactone, estragole, eugenol, oak moss, opoponax resinoid, orange oil, eugenol, aurantiol, galaxolide, carvacrol, camphor, carrot seed oil, clove oil, methyl cinnamate, geraniol, geranyl nitrile, isobornyl acetate, geranyl acetate, dimethyl benzyl carbinyl acetate, styralyl acetate, cedryl acetate, terpinyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, citral, citronellol, citronellal, cinnamon bark oil, styrax resinoid, cedar wood oil, cedrene, cedrol, celery seed oil, thyme oil, damascones, damascenones, thymol, tuberose absolute, terpineol, γ-terpinene, triplal, vanilla absolute, vanillin, basil oil, patchouli oil, hydroxycitronellal, α-pinene, piperitone, Peru balsam, vetiver oil, vetiverol, peppermint oil, pepper oil, heliotropin, bergamot oil, benzyl benzoate, borneol, myrrh resinoid, musk ketone, methyl nonyl acetaldehyde, γ-methylionone, menthol, L-menthol, L-menthone, eucalyptus oil, β-ionone, lime oil, lavender oil, D-limonene, linalool, lyral, lilial, lemon oil, rose absolute, rose oxide, rose oil and rosemary oil, and it is possible to use one or more of these. The concentration of the fragrance is preferably 0.001 to 5 mass%, and more preferably 0.01 to 3 mass%, relative to the total quantity of the hair treatment cosmetic preparation.

Examples of ultraviolet radiation absorbers include 2,4-dihydroxybenzophenone, 5,5'-methylene-bis(2-hydroxy-4-methoxybenzophenone), 2-(2-hydroxy-5-methylphenyl)benzotriazole, 2-(2-hydroxy-5-tert-octylphenyl)benzotriazole, 2-(2-hydroxy-3,5-di-tert-butylphenyl)-5-chlorobenzotriazole, 2-(2-hydroxy-3-tert-butyl-5-methylphenyl)-5-chlorobenzotriazole, 2-(2-hydroxy-3,5-dicumylphenyl)benzotriazole, 2,2'-methylene-bis(4-tert-octyl-6-benzotriazolylphenol), polyethylene glycol esters of 2-(2-hydroxy-3-tert-butyl-5-carboxyphenyl)benzotriazole, 2-[2-hydroxy-3-(2-acryloyloxyethyl)-5-methylphenyl]benzotriazole, 2-[2-hydroxy-3-(2-methacryloyloxyethyl)-5-tert-butylphenyl]benzotriazole, 2-[2-hydroxy-3-(2-methacryloyloxyethyl)-5-tert-octylphenyl]benzotriazole, 2-[2-hydroxy-3-(2-methacryloyloxyethyl)-5-tert-butylphenyl]-5-chlorobenzotriazole, 2-[2-hydroxy-5-(2-methacryloyloxyethyl)phenyl]benzotriazole, 2-[2-hydroxy-3-tert-butyl-5-(2-methacryloyloxyethyl)phenyl]benzotriazole, 2-[2-hydroxy-3-tert-amyl-5-(2-methacryloyloxyethyl)phenyl]benzotriazole, 2-[2-hydroxy-3-tert-butyl-5-(3-methacryloyloxypropyl)phenyl]-5-chlorobenzotriazole, 2-[2-hydroxy-4-(2-methacryloyloxymethyl)phenyl]benzotriazole, 2-[2-hydroxy-4-(3-methacryloyloxy-2-hydroxypropyl)phenyl]benzotriazole, 2-[2-hydroxy-4-(3-methacryloyloxypropyl)phenyl]benzotriazole, phenyl salicylate, resorcinol monobenzoate, 2,4-di-tert-butylphenyl-3,5-di-tert-butyl-4-hydroxybenzoate, octyl(3,5-di-tert-butyl-4-hydroxy)benzoate, dodecyl(3,5-di-tert-butyl-4-hydroxy)benzoate, tetradecyl(3,5-di-tert-butyl-4-hydroxy)benzoate, hexadecyl(3,5-di-tert-butyl-4-hydroxy)benzoate, octadecyl(3,5-di-tert-butyl-4-hydroxy)benzoate, behenyl (3,5-di-tert-butyl-4-hydroxy)benzoate, 2-ethyl-2'-ethoxyoxanilide, 2-ethoxy-4'-dodecyloxanilide, ethyl-α-cyano-β,β-diphenylacrylate, methyl-2-cyano-3-methyl-3-(p-methoxyphenyl)acrylate, ethylhexyl methoxycinnamate, bis-ethylhexyloxyphenol methoxyphenyl triazine, hexyl diethylaminohydroxybenzoylbenzoate and a variety of metal salts and metal chelates, and it is possible to use one or more of these. The concentration of the ultraviolet radiation absorber is preferably 0.001 to 10 mass%, and more preferably 0.01 to 5 mass%, relative to the total quantity of the hair treatment cosmetic preparation.

Examples of solvents include ethanol, isopropyl alcohol, butanol, isobutyl alcohol, acetone, ethyl acetate, ethylene glycol monoethyl ether and water, and it is possible to use one or more of these. The concentration of the solvent is preferably 10 to 99 mass%, and more preferably 20 to 95 mass%, relative to the total quantity of the hair treatment cosmetic preparation.

The method for producing a polyurethane for cosmetic preparations of this invention comprises preparing a polyurethane prepolymer by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group in the molecule and has a molecular weight of 100 to 300, and (D) a diisocyanate containing at least one compound selected from the group consisting of isophorone diisocyanate, hexamethylene diisocyanate and dicyclohexylmethane-4,4'-diisocyanate; and then reacting this polyurethane prepolymer with a chain extender (E) which contains at least one compound selected from the group consisting of water, ethylene diamine and propylene diamine. Moreover, the method for producing the polyurethane prepolymer is not particularly limited, and a well-known method can be used. For example, it is possible to use a prepolymer mixing method consisting of reacting the polyester polyol (A), the aliphatic diol (B), the carboxyl group-containing diol (C) and the diisocyanate (D) and, if necessary, adding to, and dispersing in, a solvent containing a neutralizing agent and an emulsifier.

Usage quantities of materials used in the production of the polyurethane prepolymer of this invention are not particularly limited, but from the perspectives of adjusting the molecular weight of the obtained polyurethane for cosmetic preparations and achieving a balance of properties, these usage quantities are such that the ratio of the number of moles of hydroxyl groups in the polyester polyol (A), the number of moles of hydroxyl groups in the aliphatic diol (B) and the number of moles of hydroxyl groups in the carboxyl group-containing diol (C) ((A):(B):(C)) is preferably 1:0.5-2.0:0.5-6.0, more preferably 1:0.6-1.8:0.8-5.0, and further preferably 1:0.7-1.5:1.0-4.5, if the number of moles of hydroxyl groups in the polyester polyol (A) is taken to be 1.

In addition, from the perspectives of adjusting the molecular weight of the obtained polyurethane for cosmetic preparations and achieving a balance of properties, usage quantities of the materials are such that if the total number of moles of hydroxyl groups in the polyester polyol (A), hydroxyl groups in the aliphatic diol (B) and hydroxyl groups in the carboxyl group-containing diol (C) is taken to be 1 mole, the number of moles of isocyanate groups contained in the diisocyanate (D) is preferably 1.05 to 2.00 moles, more preferably 1.10 to 1.60 moles, and further preferably 1.15 to 1.40 moles.

In addition, from the perspectives of adjusting the molecular weight of the obtained polyurethane for cosmetic preparations and achieving a balance of properties, usage quantities of the materials are such that the ratio of the number of moles of hydroxyl groups in the polyester polyol (A), the number of moles of hydroxyl groups in the aliphatic diol (B), the number of moles of hydroxyl groups in the carboxyl group-containing diol (C) and the number of isocyanate groups in the diisocyanate (D) ((A):(B):(C):(D)) is preferably 1:0.5-2.0:0.5-6.0:2.5-10.0, more preferably 1:0.6-1.8:0.8-5.0:3.5-9.0, further preferably 1:0.7-1.5:1.0-4.5:4.5-8.0, and further preferably 1:0.8-1.4:2.0-4.0:5.0-7.5, if the number of moles of hydroxyl groups in the polyester polyol (A) is taken to be 1.

In addition, in cases where the polyurethane prepolymer is reacted with the chain extender (E) that contains at least one compound selected from the group consisting of water, ethylene diamine and propylene diamine, usage quantities are such that the total number of moles of water molecules and amino groups in the chain extender (E) is preferably at least 1.0 times the number of moles of isocyanate groups remaining in the polyurethane prepolymer (a number of moles obtained by subtracting the number of moles of hydroxyl groups in the polyester polyol (A), hydroxyl groups in the aliphatic diol (B) and hydroxyl groups in the carboxyl group-containing diol (C) from the number of moles of isocyanate groups in the diisocyanate (D)). In this invention, by extending the molecular chain of the polyurethane prepolymer by means of the chain extender, the molecular weight of the obtained polyurethane for cosmetic preparations can be easily adjusted within a desired range. Moreover, in cases where water is used as the chain extender (E), the water can be used as a solvent for the obtained polyurethane for cosmetic preparations, and it is therefore possible to obtain aqueous solutions or dispersions of the polyurethane for cosmetic preparations having a variety of concentrations by adjusting the usage quantity of the water.

The reactions mentioned above are not particularly limited as long as conditions are such that the raw materials react, and it is possible to carry out the reactions either by introducing the entire amount of raw materials all at once or by dividing the raw materials into several portions and introducing the portions separately. For example, a specific example is a method comprising introducing raw materials of the components into a reaction system, either all at once or as several separate portions, mixing at a temperature of 30 to 160°C, and preferably 40 to 160°C, and at increased pressure, reduced pressure or normal pressure, and maintaining this state for a period of 30 minutes to 10 hours until the reaction is complete.

In the method for producing a polyurethane for cosmetic preparations of this invention, neutralizing agents, emulsifiers and solvents may be used if necessary. Examples of neutralizing agents include ammonia, monofunctional organic amines such as trimethylamine, triethylamine, tripropylamine, tributylamine, N-methyldiethanolamine and triethanolamine, and inorganic bases such as sodium hydroxide, potassium hydroxide and ammonia, and these can be used at a quantity sufficient for neutralizing carboxyl groups. Moreover, in cases where a neutralizing agent is used, the neutralizing agent may be added to the polyurethane prepolymer prior to the reaction with the chain extender, at the same time as the polyurethane prepolymer is reacted with the chain extender, or after the polyurethane prepolymer is reacted with the chain extender. From the perspectives of adjusting the molecular weight of the polyurethane for cosmetic preparations and achieving a balance of properties, It is preferable to add the neutralizing agent to the polyurethane prepolymer before the reaction with the chain extender or at the same time as this reaction, and it is more preferable to use an inorganic base as the neutralizing agent in this case.

Examples of emulsifiers include well-known anionic surfactants, non-ionic surfactants, cationic surfactants, and mixtures of these surfactants. Examples of solvents include ethanol, propanol, butanol, hexane, toluene, ethyl acetate, butyl acetate and water.

In addition, it is possible to use a catalyst in the reactions mentioned above in order to facilitate the reactions.

Examples of catalysts include strong acids such as sulfuric acid and toluene sulfonic acid; metal halides such as titanium tetrachloride, hafnium chloride, zirconium chloride, aluminum chloride, gallium chloride, indium chloride, iron chloride, tin chloride and boron fluoride; alkali metal and alkaline metal hydroxides, alcoholates and carbonates, such as sodium hydroxide, potassium hydroxide, lithium methylate and sodium carbonate; metal oxides such as aluminum oxide, calcium oxide, barium oxide and sodium oxide; organometallic compounds such as tetraisopropyl titanate, dibutyl tin dichloride, dibutyl tin oxide; and dibutyl tin bis(2-ethylhexylthioglycolate); and soaps such as sodium acetate, potassium acetate, sodium propionate, potassium propionate, sodium octylate, potassium octylate, sodium laurate and potassium laurate. The blending quantity of these catalysts is not particularly limited, but is approximately 0.01 to 1 mass% relative to the total mass of raw materials. Moreover, although the reactions progress without use of a catalyst, use of a catalyst increases the reaction rate, and therefore achieves the advantageous effect of shortening the reaction time.

In this invention, by adjusting the ratio of the usage quantities of the materials of components (A) to (D), producing the polyurethane prepolymer and reacting with the chain extender (E), it is possible to adjust the molecular weight within a specific range and thereby obtain a polyurethane for cosmetic preparations which exhibits excellent properties.

In the method for producing a polyurethane for cosmetic preparations of this invention, other raw materials (excluding neutralizing agents) able to react with the materials of components (A) to (E) described above may be used, but from the perspective of achieving the advantageous effect of this invention, it is preferable to only react the polyester polyol (A), the aliphatic diol (B), the carboxyl group-containing diol (C), the diisocyanate (D) and the chain extender (E). More specifically, it is preferable to produce a polyurethane prepolymer from materials consisting only of components (A) to (D), and then react this prepolymer with component (E) only so as to produce the polyurethane for cosmetic preparations of this invention. Optionally, it is possible to add a neutralizing agent to the polyurethane prepolymer so as to neutralize carboxyl groups remaining in the polyurethane prepolymer.

Examples

This invention will now be explained in greater detail through the use of working examples and comparative examples, but this invention is in no way limited to these examples. Moreover, in the working examples etc. given below, % means mass% unless explicitly indicated otherwise.

<Working Example 1: Production of polyurethane 1 for cosmetic preparations>

A polyurethane prepolymer was produced by charging a glass reaction vessel having a stirrer, a condenser tube and a nitrogen inlet tube with 341.9 g of a polyester polyol having two hydroxyl groups in each molecule and a molecular weight of 1000, (ADEKA NEW ACE YG-108 produced by ADEKA Corp.), which was obtained by reacting isophthalic acid, adipic acid and hexane diol, as the polyester polyol (A), 37.4 g of neopentyl glycol as the aliphatic diol (B), 143.3 g of dimethylolpropionic acid as the carboxyl group-containing diol (C) and 477.3 g of isophorone diisocyanate as the diisocyanate (D), and carrying out a reaction for 4 hours at 80°C. At this point, the ratio of the number of moles of hydroxyl groups in the polyester polyol (A), hydroxyl groups in the aliphatic diol (B), hydroxyl groups in the carboxyl group-containing diol (C) and isocyanate groups in the diisocyanate (D) was 1:1:3:6 if the number of moles of hydroxyl groups in the polyester polyol (A) is taken to be 1. Following the reaction, 1,611.0 g of water as the chain extender (E) and a solvent and 41.0 g of potassium hydroxide as a neutralizing agent were added, a reaction was carried out for 2 hours at 40°C, and the reaction was terminated when it was confirmed by an infrared absorption spectrum that there was no NCO absorption, thereby producing polyurethane 1 for cosmetic preparations. The weight average molecular weight, as determined in terms of styrene by gel permeation chromatography (GPC) measurements, of the obtained polyurethane 1 for cosmetic preparations was 137,000.

<Comparative Example 1: Production of polyurethane 2 for cosmetic preparations>

Polyurethane 2 for cosmetic preparations was produced using a similar method to that used for producing polyurethane 1 for cosmetic preparations, except that usage quantities of raw materials were altered, the ratio of the number of moles of hydroxyl groups in the polyester polyol (A), hydroxyl groups in the aliphatic diol (B), hydroxyl groups in the carboxyl group-containing diol (C) and isocyanate groups in the diisocyanate (D) was 1:2:3:6, and the chain extender (E) was not used. The weight average molecular weight, as determined in terms of styrene by gel permeation chromatography (GPC) measurements, of the obtained polyurethane 2 for cosmetic preparations was 30,000.

<Comparative Example 2: Production of polyurethane 3 for cosmetic preparations>

A commercially available polyurethane (Luviset P.U.R produced by BASF, 30% aqueous solution) was used as polyurethane 3 for cosmetic preparations. This polyurethane is a copolymer consisting of isophthalic acid, adipic acid, hexylene glycol, neopentyl glycol, dimethylolpropionic acid and isophorone diisocyanate, and had a weight average molecular weight, as determined in terms of styrene by gel permeation chromatography (GPC) measurements, of 16,000.

<Evaluation of warm water resistance>

Polyurethanes 1 to 3 for cosmetic preparations were coated on glass sheets and dried so as to form a film of the polyurethane for cosmetic preparations at a thickness of 100 $\mu$m on each glass sheet. Next, each glass sheet on which a film had been formed was immersed for 30 seconds in warm water at a temperature of 40°C and then removed from the warm water, after which the state of the film was observed after drying for 12 hours at room temperature. The results showed that the state of the film formed using polyurethane 1 for cosmetic preparations was maintained, but films formed using polyurethane 2 for cosmetic preparations and polyurethane 3 for cosmetic preparations completely dissolved in the warm water, and no film remained. Therefore, it was understood that the polyurethane for cosmetic preparations of this invention exhibits excellent warm water resistance.

<Production of hair treatment agents>

Hair treatment agents 1 to 6 having formulations shown in Table 1 below (% denotes mass%) were produced using polyurethanes 1 to 3 for cosmetic preparations. Moreover, the blending quantity of a polyurethane for cosmetic preparations in a hair treatment agent indicates the amount of the polyurethane for cosmetic preparations excluding solvents such as water.

[Table 1]

| | | Hair treatment agent 1 | Hair treatment agent 2 | Hair treatment agent 3 | Hair treatment agent 4 | Hair treatment agent 5 | Hair treatment agent 6 |
|---|---|---|---|---|---|---|---|
| Hair treatment agent | Polyurethane 1 for cosmetic preparations | 10% | 1% | | | | |
| | Polyurethane 2 for cosmetic preparations | | | 10% | 1% | | |
| | Polyurethane 3 for cosmetic preparations | | | | 10% | 1% | |
| | Ethanol | 52% | 52% | 52% | 52% | 52% | 52% |
| | Fragrance | 0.05% | 0.05% | 0.05% | 0.05% | 0.05% | 0.05% |
| | Water | Balance | Balance | Balance | Balance | Balance | Balance |
| Evaluation | Smoothness | ∘ | ∘ | × | × | × | × |
| | Curl retention ratio | 87% | 38% | 45% | 8% | 53% | 7% |

<Evaluation of smoothness and curl retention ratio>

1 g of each of hair treatment agents 1 to 6 were applied to 3 g bundles of hair (having a length of 35 cm) and then dried for 1 hour at 50°C, after which the smoothness of each hair bundle was evaluated using the evaluation criteria described below. In addition, curl retention ratio was evaluated for each hair bundle using the evaluation method described below. Evaluation results are shown in Table 1.

(Evaluation criteria for smoothness)

∘: Smooth and soft feel

×: No soft or smooth feel

(Method for evaluating curl retention ratio)

Curls were formed in each of the dried hair bundles using a curler having a curl diameter of 2 cm, after which each hair bundle was suspended on a board having a marked scale, and after drying for 1 hour at 50°C, the length ($L_1$) of each hair bundle was measured. Next, each curled hair bundle was placed in a constant temperature chamber set to a temperature of 30°C and relative humidity of 90% in a state whereby the hair bundle was suspended on the board having a marked scale, and left to rest for 1 hour. After being left to rest for 1 hour, the length ($L_2$) of each hair bundle was measured, and the curl retention ratio (%) was determined using the formula shown below. Moreover, as the curl retention ratio approaches 100%, the curl retention power increases, which indicates that the hair treatment agent exhibits excellent curl retention power and moisture resistance, and a curl retention ratio of 30% or more indicates practicality.

$$\texttt{Curl retention ratio (\%) = \{(35-L_2)/(35-L_1)\} \times 100}$$

<Evaluation of shape memory>

In addition, hair bundles treated with hair treatment agents 1 and 3 were evaluated in terms of shape memory. Specifically, 1 g each of hair treatment agents 1 and 3 were applied to 3 g bundles of hair (having a length of 35 cm), and dried for 1 hour at 50°C, after which curls were formed using a curler having a curl diameter of 2 cm, each hair bundle was suspended on a board having a marked scale, and after drying for 1 hour at 50°C, the length ($L_3$) of each hair bundle was measured. Next, a load of 100 g was suspended from the bottom of each curled hair bundle for 1 minute, 3 minutes, 5 minutes and 20 minutes, after which the length ($L_4$) of each hair bundle was measured, and shape memory values for different loading times were calculated using the formula shown below. Results are shown in Table 2. Moreover, as the shape memory value approaches 100% the curl retention power against loading increases, which indicates that the hair treatment agent exhibits excellent curl retention power. In addition, a loading time of 0 minutes corresponds to the length of the hair bundle before the load was suspended.

$$\texttt{Shape memory value (\%) = \{(35-L_4)/(35-L_3)\} \times 100}$$

[Table 2]

| | Hair treatment agent used | Loading time | | | | |
|---|---|---|---|---|---|---|
| | | 0 min | 1 min | 3 min | 5 min | 20 min |
| Shape memory value | Hair treatment agent 1 | 100% | 78% | 66% | 56% | 34% |
| | Hair treatment agent 3 | 100% | 38% | 32% | 30% | 14% |

As can be understood from the results shown above, the polyurethane for cosmetic preparations of this invention exhibits excellent warm water resistance and exhibits excellent smoothness and curl retention power when used as a hair treatment agent. Moreover, conventional polyurethanes produced in the comparative examples were able to increase the curl retention ratio when incorporated at a high concentration in a hair treatment agent, but did not exhibit smoothness and were not suitable for use as cosmetic preparations. Therefore, the polyurethane for cosmetic preparations of this invention exhibits excellent properties such as coating film flexibility, strength and stability, and exhibits excellent properties when used in cosmetic preparations.

**Claims**

1. A polyurethane for cosmetic preparations, which is obtained by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group and two hydroxyl groups in the molecule and has a molecular weight of 100 to 300, (D) a diisocyanate containing at least one compound selected from the group consisting of isophorone diisocyanate, hexamethylene diisocyanate and dicyclohexylmethane-4,4'-diisocyanate, and (E) a chain extender which contains at least one compound selected from the group consisting of water, ethylene diamine and propylene diamine and which has a weight average molecular weight of 50,000 to 300,000.

2. The polyurethane for cosmetic preparations according to claim 1, wherein the polyester polyol (A) is obtained by reacting one or more types of polycarboxylic acid selected from the group consisting of phthalic acid, isophthalic acid, terephthalic acid and adipic acid with one or more types of aliphatic polyol selected from the group consisting of ethylene glycol, diethylene glycol, butane diol, neopentyl glycol, hexane diol and hexylene glycol.

3. The polyurethane for cosmetic preparations according to claim 1 or claim 2, which is obtained by reacting materials consisting of the polyester polyol (A), the aliphatic diol (B), the carboxyl group-containing diol (C), the diisocyanate (D) and the chain extender (E), wherein the diisocyanate (D) consists of at least one type selected from the group consisting of isophorone diisocyanate, hexamethylene diisocyanate and dicyclohexylmethane-4,4'-diisocyanate, and the chain extender (E) consists of at least one type selected from the group consisting of water, ethylenediamine and propylenediamine.

4. The polyurethane for cosmetic preparations according to any one of claims 1 to 3, wherein carboxyl groups in the polyurethane for cosmetic preparations are neutralized.

5. A cosmetic preparation comprising the polyurethane for cosmetic preparations according to any one of claims 1 to 4.

6. The cosmetic preparation according to claim 5, which is used for treating hair.

7. A method for producing a polyurethane for cosmetic preparations, the method comprising a step of preparing a polyurethane prepolymer by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group in the molecule and has a molecular weight of 100 to 300, and (D) a diisocyanate containing at least one compound selected from the group consisting of isophorone diisocyanate, hexamethylene diisocyanate and dicyclohexylmethane-4,4'-diisocyanate; and a step of reacting this polyurethane prepolymer with a chain extender (E) which contains at least one compound selected from the group consisting of water, ethylene diamine and propylene diamine.

8. The method for producing a polyurethane for cosmetic preparations according to claim 7, wherein if the total number of moles of hydroxyl groups contained in the polyester polyol (A), the aliphatic diol (B) and the carboxyl group-containing diol (C) is taken to be 1 mole, the number of moles of isocyanate groups contained in the diisocyanate (D) is 1.05 to 2.00.

9. The method for producing a polyurethane for cosmetic preparations according to claim 7 or claim 8, wherein the ratio of the number of moles of hydroxyl groups in the polyester polyol (A), the number of moles of hydroxyl groups in the aliphatic diol (B) and the number of moles of hydroxyl groups in the carboxyl group-containing diol (C) ((A):(B):(C)) is 1:0.5-2.0:0.5-6.0 if the number of moles of hydroxyl groups in the polyester polyol (A) is taken to be 1.

10. The method for producing a polyurethane for cosmetic preparations according to any one of claims 7 to 9, comprising a step of adding a neutralizing agent after preparing the polyurethane prepolymer.

11. The method for producing a polyurethane for cosmetic preparations according to any one of claims 7 to 9, the method consisting of a step of preparing a polyurethane prepolymer by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group in the molecule and has a molecular weight of 100 to 300, and (D) a diisocyanate containing at least one compound selected from the group consisting of isophorone diisocyanate, hexamethylene diisocyanate and dicyclohexylmethane-4,4'-diisocyanate; and a step of reacting this polyurethane prepolymer with a chain extender (E) which contains at least one compound selected from the group consisting of water, ethylene diamine and propylene diamine.

12. The method for producing a polyurethane for cosmetic preparations according to any one of claims 7 to 9, the method consisting of a step of preparing a polyurethane prepolymer by reacting (A) a polyester polyol having two or more hydroxyl groups in each molecule, (B) an aliphatic diol consisting of two hydroxyl groups and an aliphatic hydrocarbon group having 3 to 8 carbon atoms, (C) a carboxyl group-containing diol which has a carboxyl group in the molecule and has a molecular weight of 100 to 300, and (D) a diisocyanate containing at least one compound selected from the group consisting of isophorone diisocyanate, hexamethylene diisocyanate and dicyclohexylmethane-4,4'-diisocyanate; a step of reacting this polyurethane prepolymer with a chain extender (E) which contains at least one compound selected from the group consisting of water, ethylene diamine and propylene diamine; and a step of adding a neutralizing agent.

13. Use of the polyurethane for cosmetic preparations according to any one of claims 1 to 4 in the production of a cosmetic preparation.

14. A hair dressing method consisting of applying the cosmetic preparation according to claim 6 to hair.

## INTERNATIONAL SEARCH REPORT

International application No.

PCT/JP2020/005985

### A. CLASSIFICATION OF SUBJECT MATTER
C08G 18/08(2006.01)i; A61K 8/87(2006.01)i; A61Q 5/06(2006.01)i
FI: C08G18/08 019; A61K8/87; A61Q5/06

According to International Patent Classification (IPC) or to both national classification and IPC

### B. FIELDS SEARCHED

Minimum documentation searched (classification system followed by classification symbols)
C08G18/00-18/87; A61K8/00-8/99; A61Q1/00-99/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2004-504464 A (NOVEON IP HOLDINGS CORP.) 12.02.2004 (2004-02-12) claims, paragraph [0030], column "examples", etc. | 1–14 |
| X | WO 2012/114833 A1 (DIC CORPORATION) 30.08.2012 (2012-08-30) claims, paragraphs [0013], [0063], [0114], column "examples", etc. | 1–14 |
| X | JP 2009-535382 A (BASF SE) 01.10.2009 (2009-10-01) claims, paragraphs [0160], [0195], [0257], column "examples", etc. | 1–14 |

☒ Further documents are listed in the continuation of Box C.    ☒ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 24 April 2020 (24.04.2020) | 12 May 2020 (12.05.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2020/005985 |

C (Continuation).   DOCUMENTS CONSIDERED TO BE RELEVANT

| Category* | Citation of document, with indication, where appropriate, of the relevant passages | Relevant to claim No. |
|---|---|---|
| X | JP 11-310520 A (NATIONAL STARCH AND CHEMICAL INVESTMENT HOLDING CORPORATION) 09.11.1999 (1999-11-09) claims, paragraphs [0019]-[0021], column "examples" (for example, example 23), etc. | 1-14 |
| A | JP 11-228363 A (NIPPON NSC LTD.) 24.08.1999 (1999-08-24) | 1-14 |

Form PCT/ISA/210 (continuation of second sheet) (January 2015)

## INTERNATIONAL SEARCH REPORT
Information on patent family members

International application No.

PCT/JP2020/005985

| Patent Documents referred in the Report | Publication Date | Patent Family | Publication Date |
|---|---|---|---|
| JP 2004-504464 A | 12 Feb. 2004 | US 2002/0028875 A1 claims, paragraph [0033], column "examples", etc. WO 2002/008327 A1 | |
| WO 2012/114833 A1 | 30 Aug. 2012 | US 2013/0331511 A1 claims, paragraphs [0013], [0063], [0114], column "examples", etc. EP 2679614 A | |
| JP 2009-535382 A | 01 Oct. 2009 | US 2009/0257960 A1 claims, paragraphs [0228], [0275], [0343], column "examples", etc. WO 2007/128776 A1 | |
| JP 11-310520 A | 09 Nov. 1999 | US 5968494 A claims, column 4, column "examples" (for example, example 23), etc. EP 937451 A2 | |
| JP 11-228363 A | 24 Aug. 1999 | WO 1999/039688 A2 | |

Form PCT/ISA/210 (patent family annex) (January 2015)

**EP 3 929 229 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H05262622 B
- JP 2003137730 A
- WO 07509741 A
- WO 2008035563 A
- JP 2016117715 A